# FASCICULE DE BREVET EUROPEEN

(11) **EP 2 664 354 B1**
(45) Date de publication et mention de la délivrance du brevet: **16.09.2015**
(21) Numéro de dépôt: 13167517.5
(22) Date de dépôt: 13.05.2013
(51) Int. Cl.: A61N 1/05

(54) **Sonde médicale avec une électrode en forme de bague destinée à être implantée dans un vaisseau cardiaque ou cérébral et un procédé pour sa fabrication**
Medizinische Leitung mit einer Ringelektrode zur Implantation in ein Herz- oder Hirngefäß und ein Verfahren zu deren Herstellung
Medical lead with a ring electrode for implantation in a cardiac or cerebral blood vessel and a method for its manufacture

(30) Priorité: 16.05.2012 FR 1254548
(43) Date de publication de la demande: 20.11.2013
(73) Titulaire: Sorin CRM SAS, 92140 Clamart Cedex (FR)
(72) Inventeur: Ollivier, Jean-François, 91190 Villiers le Bacle (FR); Shan, Nicholas, 91260 Juvisy sur Orge (FR); D'Hiver, Philippe, 92320 Chatillon (FR)
(74) Mandataire: Dupuis-Latour, Dominique

(56) Documents cités:
- US-A- 6 018 684
- US-A1- 2005 113 896
- US-A1- 2006 229 693
- US-A1- 2006 265 037
- US-A1- 2009 134 134

## Description

L'invention concerne de façon générale les "dispositifs médicaux implantables actifs" tels que définis par la directive 90/385/CEE du 20 juin 1990 du Conseil des communautés européennes.

Elle concerne plus précisément une microsonde de détection/stimulation destinée à être implantée dans des réseaux veineux, artériels ou lymphatiques.

Une telle sonde peut notamment être utilisée en cardiologie, en particulier pour être implantée dans le réseau veineux du sinus coronaire afin de stimuler une cavité gauche ou droite du coeur.

Les microsondes sont cependant utiles dans de nombreuses autres applications médicales, chaque fois que l'on est en présence d'un réseau veineux, artériel ou même lymphatique, comme notamment le réseau cérébral veineux ou artériel.

En particulier, la stimulation électrique a permis des avancées importantes en neurologie dans le domaine de la neuromodulation, technique qui consiste à stimuler des zones cibles du cerveau pour le traitement de troubles tels que la maladie de Parkinson, l'épilepsie et autres maladies neurologiques. Une telle technique permet une approche moins invasive de ces traitements et surtout une efficacité supérieure des traitements administrés.

La difficulté dans ces applications est d'atteindre des régions peu accessibles aujourd'hui, grâce à des "microsondes" de stimulation de très petit diamètre mais présentant néanmoins une très grande robustesse afin de garantir la biostabilité à long terme.

Ainsi, la taille des sondes implantables actuelles étant typiquement de l'ordre de 4 à 6 French (1,33 à 2 mm), il serait souhaitable de pouvoir réduire ce diamètre à moins de 2 French (0,66 mm). Une telle taille de microsonde permettrait notamment d'atteindre des veinules de très petite dimension, inaccessibles aujourd'hui avec des dispositifs de taille supérieure. Une telle microsonde doit également pouvoir naviguer facilement dans les réseaux veineux artériels ou lymphatiques en présentant une souplesse suffisante pour pouvoir être introduite dans des réseaux de vaisseaux présentant de fortes tortuosités, d'anastomoses.

Cependant, il est clair que la réduction de diamètre des sondes augmente leur complexité technologique et impose des contraintes techniques génératrices de risques.

La microsonde comprend un conducteur central destiné à être relié au générateur de l'implant, ce conducteur étant revêtu d'une gaine électriquement isolante. Elle présente en outre sur sa partie active une ou plusieurs électrodes de détection/stimulation électriquement reliées au conducteur central, et destinées à venir en contact avec la paroi du vaisseau cible.

L'invention vise plus précisément la réalisation des électrodes de détection/stimulation d'une telle microsonde.

Une première technique de réalisation des électrodes consiste à percer ponctuellement la gaine isolante de façon à mettre à nu le microcâble en un ou plusieurs points. Les parties dénudées vont constituer ensemble un réseau d'électrodes reliées en série (configuration de sonde monopolaire), permettant de multiplier les points de stimulation et d'assurer ainsi une diffusion multizone de l'énergie de stimulation délivrée par l'implant.

Cette technique est par exemple décrite dans le EP 2 455 131 A1 (publié le 23.05.2012, priorité du 19.11.2010), au nom de la Demanderesse.

Cette même demande propose également, en variante, de réaliser la partie active de microsonde en enfilant successivement et alternativement sur le microcâble des tubes isolants et des électrodes conductrices courtes en platine iridié, en forme de bagues. Les tubes isolants, par exemple en polyuréthane, sont collés sur le microcâble et les électrodes en platine iridié sont serties directement sur ce microcâble.

Une autre technique encore consiste à appliquer sur le microcâble une enduction de colle polyuréthane isolante, en laissant apparaitre localement en réserve des surfaces non revêtues, conductrices.

Avec les techniques qui prévoient la mise à nu du microcâble (ablation de l'isolant ou surfaces laissées en réserve), il est toutefois nécessaire de prévoir un revêtement conducteur, par exemple un alliage de nitrure de titane ou un dépôt de carbone de type *Carbofilm* (marque déposée) par une technique de pulvérisation cathodique telle que celle exposée notamment dans les US 5 370 684 A et US 5 387 247 (tous deux au nom de Sorin Biomedica SpA), pour protéger des phénomènes de corrosion le câble mis à nu.

En effet, celui-ci est réalisé en un alliage tel que le MP35N (35% Ni, 35% Co, 20% Cr et 10% Mo), considéré comme inoxydable dans des conditions standard, choix nécessité par les contraintes particulièrement sévères de robustesse mécanique et de tenue à la fatigue sur le long terme imposées au microcâble. Or un tel matériau se révèle assez sensible à l'électrocorrosion, c'est-à-dire à un phénomène de corrosion accentué par la circulation du courant dans des zones polaires (électrodes) et par le contact avec les fluides organiques environnants (sang).

Il convient donc d'éviter tout risque de perfusion des fluides corporels vers le microcâble. Pour cette raison, le microcâble doit être parfaitement isolé de tout contact avec l'environnement de la microsonde, d'où le revêtement conducteur additionnel de nitrure de titane NiTi ou de carbone sur les zones d'électrode ou bien le sertissage sur le microcâble de bagues en platine iridié (matériau noble, insensible à la corrosion).

Cette contrainte d'isolement du microcâble avec le milieu extérieur, notamment dans la région des électrodes, doit être respectée i) sur toute la durée de vie escomptée de la microsonde, soit dix ans, et ii) en dépit de toutes les sollicitations mécaniques que la microsonde est susceptible de subir, typiquement 400 000 000 de sollicitations en flexion sans rupture, ce chiffre correspondant au nombre moyen de battements cardiaques sur la durée de vie de la microsonde. Le respect de ces conditions est indispensable pour que la microsonde puisse être implantée de façon permanente dans le corps, typiquement dans un vaisseau coronaire.

La durée de vie est de ce fait un paramètre fondamental, qui doit être absolument pris en compte lors de la conception d'une microsonde de stimulation. En effet, les battements du coeur et le mouvement des organes induisent sur ce type de dispositif des déformations de flexion qui doivent être parfaitement maîtrisées. En particulier, une microsonde de stimulation par le réseau veineux peut être le siège de déformations sous des courbures très supérieures à celles que subit une sonde conventionnelle, dans la mesure où elle doit suivre les déformations des veines. Ceci provoque des contraintes plus importantes et rend sa résistance à la fatigue plus contraignante.

Le US 2009/134134 A1 décrit une structure de sonde comportant une électrode annulaire reliée à un conducteur spiralé noyé dans l'épaisseur de la gaine creuse du corps de sonde. La gaine isolante est dénudée localement pour faire apparaitre le conducteur, et une la bague est soudée à ce dernier pour établir la liaison électrique. Mais le risque de perfusion à long terme des fluides corporels vers le conducteur reste entier, car il n'est prévu aucun moyen spécifique de protection de la soudure ni d'isolement du conducteur avec l'environnement de la microsonde.

Les US 2006/265037 A, US 2005/113896 A1 et US 6 018 684 A1 décrivent d'autres structures d'électrodes annulaires, présentant le même inconvénient.

Le problème de l'invention est de réaliser une microsonde dans laquelle la fonction de barrière de protection du microcâble contre l'électrocorrosion au niveau des électrodes puisse être garantie dans les conditions exposées ci-dessus, et réalisée de façon industriellement simple, économique et fiable.

Selon un premier aspect, l'invention propose un procédé de fabrication d'une sonde de détection/stimulation, procédé qui comprend les étapes suivantes : obtention d'un conducteur avec une gaine en matériau polymère électriquement isolant couvrant le conducteur sur sa périphérie et sur sa longueur, le diamètre externe de l'ensemble étant au plus égal à 2 French (0,66 mm) ; obtention d'au moins une électrode de détection/stimulation formée par une bague métallique, la bague comprenant deux parties d'extrémité s'étendant en direction longitudinale de part et d'autre d'une partie centrale ; et réalisation d'une prise de contact du conducteur avec la bague.

De façon caractéristique, l'étape de réalisation d'une prise de contact du conducteur avec la bague comprend un sertissage de la bague sur le conducteur, ce conducteur étant un microcâble de coeur plein et qui est entouré par ladite gaine en matériau polymère électriquement isolant. Le sertissage est effectué dans une région essentiellement non dénudée du microcâble, et il est opéré dans la partie centrale de la bague de manière à : i) pincer la gaine entre la bague et le microcâble ; ii) percer localement l'épaisseur de la gaine avec une zone de la face interne de la bague, de façon à mettre en contact de liaison mécanique et électrique la bague avec le microcâble dans au moins une zone de ponction de la gaine ; et ; iii) repousser le matériau isolant dans des zones adjacentes à ladite zone de ponction de la gaine, le matériau isolant de la gaine restant interposé entre le microcâble et la bague dans des régions situées de part et d'autre de ladite zone de ponction de la gaine.

Selon un second aspect, l'invention propose une sonde de détection/stimulation fabriquée par mise en oeuvre du procédé ci-dessus.

Cette sonde comprend, de manière en elle-même connue d'après le US 2009/134134 A1 précité : un conducteur apte à être relié à un générateur de dispositif médical implantable actif ; une gaine en matériau polymère électriquement isolant, couvrant le conducteur sur sa périphérie et sur sa longueur ; et au moins une électrode de détection/stimulation formée par une bague métallique électriquement reliée au conducteur, destinée à venir en contact avec la paroi d'un vaisseau-cible dudit réseau veineux, artériel ou lymphatique.

Le diamètre externe de la sonde est au plus égal à 2 French (0,66 mm). La bague comprend deux parties d'extrémité s'étendant en direction longitudinale de part et d'autre d'une partie centrale. Dans la partie centrale, la face interne de la bague est en contact de liaison mécanique et électrique avec la surface du conducteur en au moins une zone de ponction de la gaine, et dans les parties d'extrémité, la face interne de la bague est en contact de liaison mécanique avec la surface de la gaine, avec interposition du matériau isolant de la gaine entre le conducteur et la bague dans des régions situées de part et d'autre de ladite au moins une zone de ponction de la gaine.

Le conducteur est un microcâble de coeur plein et qui est entouré par la gaine en matériau polymère électriquement isolant ; la bague est disposée sur le microcâble dans une région essentiellement non dénudée de celui-ci mais comprenant ladite au moins une zone de ponction de la gaine ; et dans la partie centrale, le matériau isolant est interposé entre la bague et le microcâble dans des zones adjacentes à ladite au moins une zone de ponction de la gaine.

Selon diverses caractéristiques subsidiaires avantageuses :
- la zone de ponction de la gaine est une zone circonférentielle continue et située dans un plan orthogonal à l'axe de la gaine, ou bien une zone circonférentielle discontinue, également située dans un plan orthogonal à l'axe de la gaine, avec une pluralité de zones de ponction de la gaine ménageant entre elles des zones de continuité de l'isolant reliant les deux parties de gaine situées de part et d'autre de la bague ;
- la bague comprend sur sa face interne au moins un relief apte à définir une zone de ponction de la gaine correspondante, qui peut être soit un relief comprenant un profil longitudinal homologue de celui d'un mors de sertissage, sans surépaisseur de la bague à l'endroit de ce relief, soit un relief comprenant une surépaisseur formée sur la face interne ou externe de la bague ;
- en direction longitudinale, ce relief ne s'étend pas jusqu'à l'extrémité de la bague ;
- la bague porte sur sa face interne une pluralité de cannelures comprenant des parties rectilignes, des parties hélicoïdales et/ou des parties longitudinales ;
- la bague porte sur sa face externe des nervures longitudinales de renfort ;
- l'épaisseur de paroi de la bague est comprise entre 20 et 100 µm ;
- la longueur de la bague est comprise entre 0,5 et 1,5 mm ;
- le matériau de la bague est un alliage de platine iridié ou de palladium, et le matériau de surface du microcâble est un alliage de type MP35N ou MP35NLT ;
- l'épaisseur de paroi de la gaine est comprise entre 5 et 50 µm ;
- le matériau polymère électriquement isolant de la gaine est choisi dans le groupe suivant : polyuréthannes (PU), polyesters (PET), polyamides (PA), polycarbonates (PC), polyimides, polymères fluorés, polyétheréther-cétone (PEEK), poly-p-xylylène (parylène), polyméthacrylate de méthyle (PMM), PTFE (polytétrafluoroéthylène), FEP (propylène perfluoré), PFA (résine de copolymère perfluoroalkoxy), THV (tétrafluoroéthylène, hexafluoropropylène, fluorure de vinylidène), PVDF (polyfluorure de vinylidène), EFEP (éthylène propylène éthylène fluoré) et ETFE (éthylène tétrafluoroéthylène).

On va maintenant décrire un exemple de réalisation de la microsonde de l'invention, en référence aux dessins annexés où les mêmes références numériques désignent d'une figure à l'autre des éléments identiques ou fonctionnellement semblables.
La Figure 1 illustre en coupe longitudinale une structure typique d'électrode de sonde monopolaire conventionnelle.
La Figure 2 illustre la structure du microcâble isolé utilisable pour la réalisation de la microsonde de l'invention.
La Figure 3 montre en perspective un exemple de bague à sertir pour la réalisation d'une électrode de détection/stimulation selon l'invention.
La Figure 4 est une coupe longitudinale d'une structure d'électrode de sonde monopolaire selon l'invention, réalisée par sertissage d'une bague telle que celle de la Figure 3 sur le microcâble de la Figure 2.
Les Figures 5a et 5b sont des vues arrachées de la bague d'électrode à sertir, montrant deux configurations possibles de cannelures internes.
Les Figures 6a, 6b et 6c sont des vues arrachées schématiques montrant trois formes de réalisation possibles de reliefs formés sur la paroi interne de la bague d'électrode selon l'invention.

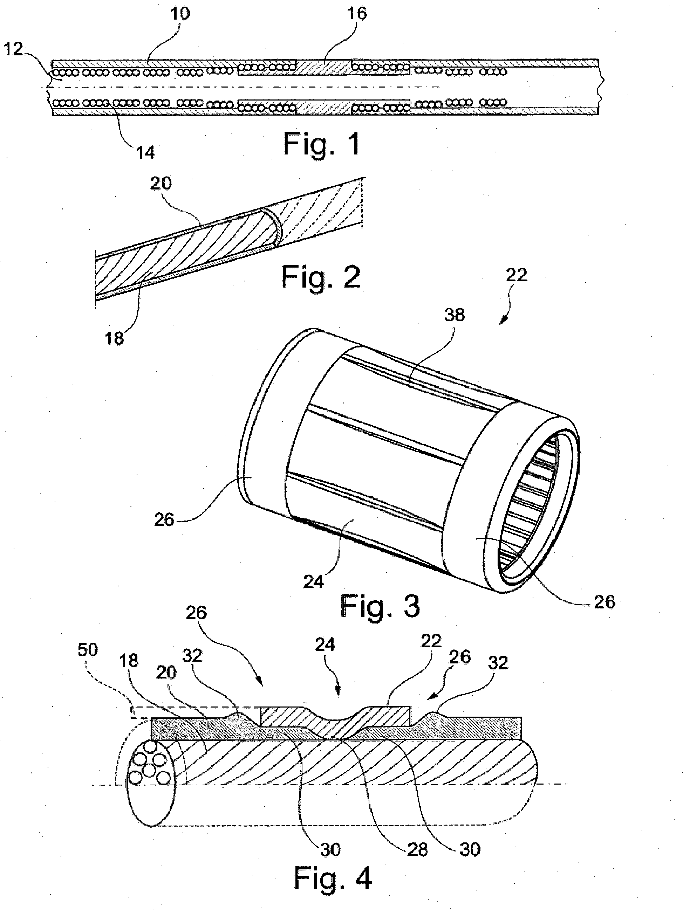

La Figure 1 illustre en coupe et de façon schématique, dans la région d'une de ses électrodes de stimulation, la structure d'une sonde monopolaire conventionnelle destinée à être implantée dans le réseau veineux coronarien.

Cette construction conventionnelle comprend un corps de sonde 10 électriquement isolant, avec une lumière centrale 12 permettant l'introduction d'un fil-guide servant à l'implantation de la sonde. La région interne de la sonde comprend également un conducteur spiralé 14 (typiquement en un alliage tel que le MP35N) électriquement relié à une électrode 16 (typiquement en alliage Ptlr) rapportée sur le corps de sonde 10. Compte tenu des contraintes de dimension du fil-guide, d'épaisseur de l'isolant, le diamètre d'une telle sonde conventionnelle est généralement compris entre 4 et 8 French (1,33 et 2,66 mm). De plus et surtout, la présence d'une électrode rapportée 16 introduit dans la structure de la sonde une discontinuité qui peut avoir une conséquence sur l'endurance à long terme de la sonde en cas de dimensionnement insuffisant des différents éléments.

L'invention concerne des microsondes dont le diamètre ne dépasse pas 2 French (0,66 mm).

Ces microsondes sont réalisées à partir d'un microcâble gainé tel que celui illustré sur la Figure 2, avec un microcâble de coeur 18 formé d'un conducteur central plein, entouré d'une couche d'isolement 20. L'ensemble présente un diamètre hors-tout au plus égal à 2 French (0,66 mm), typiquement de l'ordre de 0,5 à 2 French (0,17 à 0,66 mm).

Le microcâble est avantageusement réalisé en un alliage du type MP35N ou MP35NLT, matériau dont l'avantage essentiel est son extrême endurance en fatigue. La structure du microcâble est avantageusement une structure multifilaire se présentant sous forme d'un toron d'une pluralité de brins conducteurs de faible diamètre ; la réduction en diamètre des brins unitaires permet en effet de réduire la contrainte appliquée à chacun d'entre eux, et donc d'augmenter les performances en fatigue de la structure du toron. Une telle structure, sans lumière interne et avec plusieurs microfils torsadés ensemble, est capable d'assurer à la fois l'endurance à l'encontre des mouvements cardiaques et la résistance aux sollicitations liées à la pose.

La pose d'un tel microcâble dépourvu de lumière interne nécessite la mise en place préalable d'un corps de sonde implantable de façon permanente. Au préalable, le chirurgien dispose un cathéter principal lui permettant d'accéder au débouché du sinus coronaire, et un cathéter de sous-sélection permettant de choisir, sous amplificateur de brillance, le chemin du réseau veineux qui permettra d'atteindre la veine cible correspondant au site de stimulation choisi. Le chirurgien introduit ensuite dans ce cathéter un fil-guide, qu'il pousse pour le faire progresser à nu dans le réseau veineux coronaire de façon à sélectionner telle ou telle veine collatérale. Une fois la veine sélectionnée, le chirurgien enfile sur le fil-guide le corps de sonde, qu'il fait alors glisser et progresser sur le fil-guide jusqu'à l'emplacement choisi. Après retrait du fil-guide, le microcâble est enfilé dans le corps de sonde à partir de l'extrémité proximale de celui-ci, et il est poussé sur toute la longueur du corps de sonde jusqu'à émerger du débouché de l'extrémité proximale de ce dernier, puis est déployé au-delà de manière à le faire progresser, sous amplificateur de brillance, dans les veines collatérales jusqu'à atteindre la position souhaitée.

Ces structures de microcâble sont disponibles par exemple auprès de la Société Fort Wayne Metals Inc., Fort Wayne, USA, et sont utilisées dans le domaine médical notamment pour réaliser des conducteurs de défibrillation.

En ce qui concerne la couche d'isolement 20, celle-ci doit répondre aux caractéristiques suivantes : résistance à la fatigue ; isolement électrique ; biocompatibilité à long terme ; biostabilité ; possibilité de transformation et mise en oeuvre compatible avec le conducteur du câble de coeur.

Les matériaux pouvant être utilisés dans ce cadre sont notamment les matériaux du groupe comprenant : les polyuréthanes (PU), les polyesters (PET), les polyamides (PA), les polycarbonates (PC), les polyimides, les polymères fluorés, le polyéther-éther-cétone (PEEK), le poly-p-xylylène (parylène) et le polyméthacrylate de méthyle (PMM).

Cependant, on privilégiera les matériaux à forte inertie chimique comme les polymères fluorés, qui présentent également une très bonne qualité d'isolation. Parmi ces composés, on peut notamment citer les matériaux du groupe comprenant : le PTFE (polytétrafluoroéthylène), le FEP (propylène perfluoré), le PFA (résine de copolymère perfluoroalkoxy), le THV (tétrafluoroéthylène, hexafluoropropylène, fluorure de vinylidène), le PVDF (polyfluorure de vinylidène), l'EFEP (éthylène propylène éthylène fluoré) et l'ETFE (éthylène tétrafluoroéthylène).

Les procédés de réalisation de la couche d'isolement sur le microcâble de coeur seront fonction des matériaux utilisés, par exemple : co-extrusion sur le conducteur (pour PU, PA, PEEK, polyimides et polymères fluorés) ; dépôt par trempage dans une solution (pour PU, PA et polyimides) ; échauffement d'un tube thermorétractable (pour PET et polymères fluorés) ; dépôt chimique par voie gazeuse (pour le parylène) ; traitements plasma pour améliorer l'adhésion entre les couches.

On notera par ailleurs que, bien que l'invention ait été illustrée avec une unique couche d'un même matériau gainant le microcâble 18, il est possible de prévoir plusieurs couches successives formant la gaine 20 du microcâble, par exemple une couche de PET et une couche de ETFE.

Dans sa partie active, la microsonde est pourvue d'une ou plusieurs électrodes de détection/stimulation, électriquement reliées au conducteur central.

Comme on l'a indiqué plus haut, il s'agit au niveau de ces électrodes d'obtenir la résistance à la corrosion (le matériau du microcâble étant en effet sujet au phénomène d'électrocorrosion, tout particulièrement au niveau des pièces polaires que sont les électrodes), tout en garantissant la résistance à la fatigue impérativement requise - notamment en évitant les discontinuités de structure susceptibles de créer des points de faiblesse.

La solution proposée par l'invention consiste à sertir sur le microcâble une bague telle que celle illustrée en 22 sur la Figure 3, cette bague étant en un matériau insensible à l'électrocorrosion tel qu'un alliage de platine iridié (typiquement 90/10) ou d'un autre métal noble, tel que palladium ou tantale, insensible au phénomène d'électrocorrosion.

La longueur de la bague en direction longitudinale est typiquement de 0,5 à 1,5 mm pour un diamètre de l'ordre de 2 French (0,66 mm).

De façon caractéristique de l'invention, le sertissage de la bague est opéré directement sur le microcâble dans une région non dénudée de celui-ci, de sorte que ce sertissage perce très localement l'épaisseur de la gaine isolante 20 là où cette gaine est pincée entre la bague et le microcâble, établissant par ce biais un contact physique et électrique entre la face interne de la bague 22 et le matériau conducteur du microcâble 18.

La structure résultante obtenue après sertissage est illustrée en coupe Figure 4.

Sur la partie centrale 24 de la bague qui a subi localement le sertissage, la bague présente un diamètre réduit (sous l'effet du sertissage) par rapport aux parties d'extrémité 26 situées de part et d'autre de cette partie centrale 24. Cette géométrie résulte du sertissage différentiel de la bague, avec un effort de serrage plus élevé dans la partie centrale 24 que dans les parties d'extrémité 26. Ceci permet de créer, d'une part, le contact électrique dans la zone 28 au centre de la bague avec perçage du matériau isolant et, d'autre part, des zones de compression 30 du matériau isolant à l'entrée et à la sortie de la bague sans perçage de ce matériau, ces zones 30 assurant une étanchéité protégeant le microcâble 18 de l'électrocorrosion (lors du sertissage le matériau de la gaine est repoussé de la zone de fluage 30 vers l'extérieur en 32, à proximité des régions d'extrémité de la bague). Plus précisément :
- au droit de la partie centrale 24, la bague vient par sa face interne en contact de liaison mécanique et électrique avec les génératrices extérieures des brins à la surface du microcâble, dans la zone 28 de ponction du matériau isolant. La résistance de contact obtenue peut être très faible, inférieure à 1 Ω, et stable ;
- au droit des régions d'extrémité 26, la face interne de la bague est en contact de liaison mécanique non pas avec le microcâble, mais avec la surface de la gaine, et il y a donc dans ces régions interposition du matériau de la gaine entre le microcâble 18 et la bague 22. À cet endroit, le matériau 30 de la gaine repoussé par fluage au moment du sertissage joue le rôle de joint d'étanchéité, en formant une barrière isolante entre l'environnement extérieur et la zone de contact électrique (zone de ponction 28), et donc le matériau du microcâble, empêchant ainsi toute pénétration de fluide corporel vers la région 28 du contact électrique.

Pour pouvoir réaliser la prise de contact par sertissage direct de la bague 22 sur le microcâble 18 revêtu de sa gaine 20, l'épaisseur de paroi de la bague est choisie relativement faible, de l'ordre de 20 à 100 µm, typiquement 25 µm, en fonction des contraintes de sertissage de manière à permettre aisément une déformation suffisante, mais sans rupture de la bague.

Quant à la gaine 20, son épaisseur est choisie de l'ordre de 5 à 50 µm pour pouvoir la ponctionner facilement. Par ailleurs, le matériau de cette gaine est choisi de manière à être relativement sensible au fluage, afin de se percer dès que la pression de contact (résultant de l'effort de sertissage) est suffisamment élevée. Des matériaux tels que le ETFE et le PET répondent à cette exigence, outre leurs excellentes propriétés de biocompatibilité et de tenue à la fatigue sur le long terme.

Divers perfectionnements permettent d'optimiser la réalisation de la liaison de la bague au microcâble.

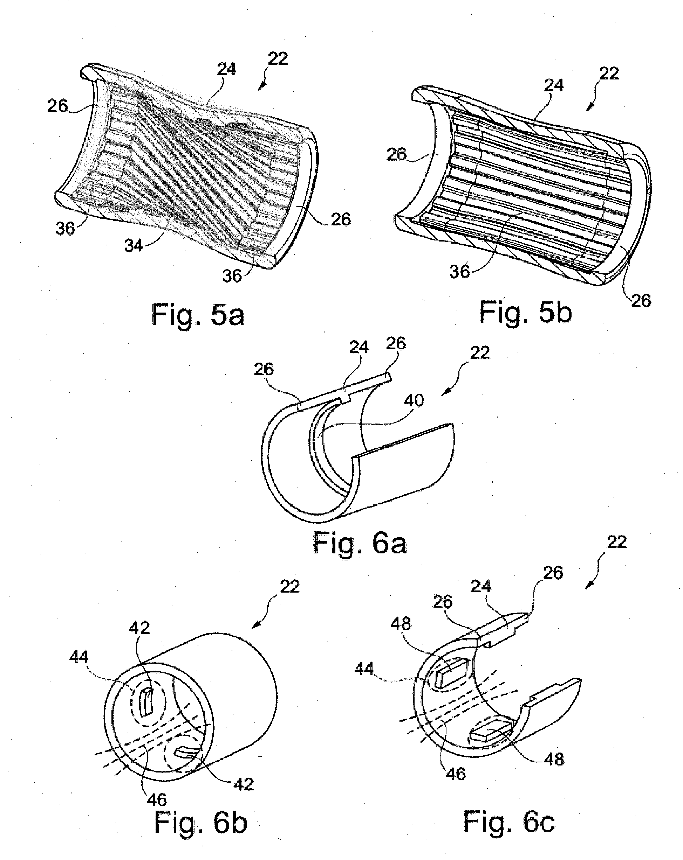

Ainsi, comme illustré Figures 5a et 5b, il est possible de donner, à l'état non serti, un profil longitudinal variable à la lumière interne de la bague, définissant un diamètre plus faible dans la partie centrale 24 que dans les parties d'extrémité 26, ceci pour favoriser le percement de l'isolant dans la partie centrale avec un outil de sertissage simple, de profil longitudinal constant. D'autre part, la réduction globale du diamètre interne dans la partie centrale 24 de la bague peut être complétée par une série de cannelures internes hélicoïdales 34 et/ou de cannelures internes rectilignes 36 dont la fonction est de favoriser le percement local de l'isolant dans la partie centrale (les parties d'extrémité 26 restant en revanche lisses et cylindriques).

Il est par ailleurs possible, comme illustré Figure 3, de renforcer le diamètre extérieur par une série de nervures longitudinales 38 de faible épaisseur, visant à maintenir la préforme interne pendant le sertissage.

Les Figures 6a, 6b et 6c illustrent des variantes de réalisation dans lesquelles le sertissage différentiel (entre la partie centrale 24 et les parties d'extrémité 26) résulte non pas d'un profil longitudinal non uniforme des mors de sertissage agissant sur la bague, et/ou d'un profil spécifique de la bague, mais de la présence d'un ou plusieurs reliefs sur la face interne de cette bague, qui peut alors conserver un profil externe rectiligne, avec une forme cylindrique de révolution permettant un sertissage avec un outil simple. En variante ou en complément, ce(s) relief(s) peu(ven)t être également formé(s) sur la face externe de la bague.

Dans l'exemple de la Figure 6a, le relief interne 40 est un relief circonférentiel continu, tandis que dans les exemples des Figures 6b et 6c il s'agit de reliefs discrets tels que 42 ou 48.

Dans ce dernier cas, chaque relief définit autour de lui une zone 44 de ponction de l'isolant, les zones de ponction 44 successives étant séparées les unes des autres par des zones intermédiaires 46 où l'isolant n'a pas été traversé. On forme ainsi des bretelles de matériau isolant intact reliant l'entrée et la sortie de la bague, ce qui a pour effet de procurer à la gaine isolante une meilleure endurance à la fatigue, du fait de la continuité mécanique de la gaine sur toute la longueur de la microsonde, y compris au niveau des électrodes.

En tout état de cause, la résistance à l'électrocorrosion de l'interface microcâble/électrode par les moyens décrits ci-dessus peut être renforcée en dotant les brins constituant le microcâble 18 d'une structure coaxiale bi-matériau, avec un coeur de brin en alliage de type MP35 associé à un revêtement d'une couche en un alliage noble, typiquement Pt-Ir, naturellement protégé contre la corrosion même dans l'hypothèse d'une infiltration accidentelle de fluide corporel.

Enfin, pour conserver à la microsonde un caractère isodiamètre, l'assemblage constitué par le microcâble 18 revêtu de sa gaine 20 et muni des bagues serties 22 peut être complété par une gaine thermorétractable supplémentaire 50 (Figure 4) disposée de part et d'autre de chaque électrode. Cette gaine thermorétractable présente le double avantage de i) permettre de donner à la microsonde à l'état fini une préforme (courbure imposée au moment du thermoformage) et ii) compenser la légère surépaisseur due au sertissage des bagues 22 sur le microcâble.

En conclusion, outre sa simplicité de conception, la solution de l'invention présente des avantages multiples :
- facilité de mise en oeuvre : on évite en effet une étape de production délicate consistant à réaliser une ablation préalable locale de l'isolant, étape qui présente des risques importants d'endommagement du microcâble ;
- protection optimale contre les risques de perfusion de fluides corporels vers le microcâble : lors du sertissage, l'isolant est repoussé à proximité immédiate des points de contact physique, renforçant alors l'étanchéité de cette zone, et évitant tout recours à un dépôt de colle silicone ou polyuréthane, qui serait très difficile au vu des très faibles dimensions des interstices à combler (espaces résiduels entre microcâble et bague sertie) ;
- possibilité d'obtention d'un profil isodiamètre, par simple ajout d'une gaine thermorétractable ;
- maintien de la continuité mécanique structurelle du produit, du fait de la continuité du microcâble, qui n'est pas interrompu comme dans les sondes conventionnelles. Du fait de sa faible longueur, l'ajout de la bague ne modifie pas sensiblement le rayon de courbure naturelle du microcâble dû à sa propre rigidité, sous un mode de contrainte correspondant à des conditions normales d'utilisation (rayon de courbure minimal de l'ordre de 4 mm, pour une longueur de bague comprise entre 0,5 et 1,5 mm) ;
- le sertissage modifiant légèrement le profil cylindrique de la surface externe de la bague, ceci a pour effet de densifier localement le courant sortant (effet de pointe électrique), avec pour conséquence une réduction du seuil de stimulation et comme avantage corrélatif une réduction corrélative de l'énergie consommée par le système.

## Revendications

1. Un procédé de fabrication d'une sonde de détection/stimulation destinée à être implantée dans un réseau veineux, artériel ou lymphatique,
ce procédé comprenant les étapes suivantes :
- obtention d'un conducteur (18) avec une gaine (20) en matériau polymère électriquement isolant couvrant le conducteur sur sa périphérie et sur sa longueur, le diamètre externe de l'ensemble étant au plus égal à 2 French (0,66 mm) ;
- obtention d'au moins une électrode de détection/stimulation formée par une bague métallique (22), la bague comprenant deux parties d'extrémité (26) s'étendant en direction longitudinale de part et d'autre d'une partie centrale (24) ; et
- réalisation d'une prise de contact du conducteur avec la bague, où
- l'étape de réalisation d'une prise de contact du conducteur avec la bague comprend un sertissage de la bague (22) sur le conducteur (18), ce conducteur étant un microcâble de coeur plein et qui est entouré par ladite gaine en matériau polymère électriquement isolant,
- le sertissage est effectué dans une région essentiellement non dénudée du microcâble, et
- le sertissage est opéré dans la partie centrale (24) de la bague procédé **caractérisé en ce que** le sertissage est opéré de manière à :
i) pincer la gaine entre la bague et le microcâble,
ii) percer localement l'épaisseur de la gaine avec une zone de la face interne de la bague, de façon à mettre en contact de liaison mécanique et électrique la bague avec le microcâble dans au moins une zone de ponction (28) de la gaine ; et
iii) repousser le matériau isolant dans des zones adjacentes à ladite zone de ponction de la gaine,
le matériau isolant repoussé de la gaine restant interposé entre le microcâble et la bague dans des régions (30) situées de part et d'autre de ladite zone de ponction de la gaine.

2. Une sonde de détection/stimulation destinée à être implantée dans un réseau veineux, artériel ou lymphatique, cette sonde comprenant :
- un conducteur (18) apte à être relié à un générateur de dispositif médical implantable actif ;
- une gaine (20) en matériau polymère électriquement isolant, couvrant le conducteur sur sa périphérie et sur sa longueur ; et
- au moins une électrode de détection/stimulation formée par une bague métallique (22) électriquement reliée au conducteur, destinée à venir en contact avec la paroi d'un vaisseau-cible dudit réseau veineux, artériel ou lymphatique,
et dans laquelle :
- le diamètre externe de la sonde est au plus égal à 2 French (0,66 mm) ;
- la bague comprend deux parties d'extrémité (26) s'étendant en direction longitudinale de part et d'autre d'une partie centrale (24) ;
- dans la partie centrale (24), la face interne de la bague est en contact de liaison mécanique et électrique avec la surface du conducteur (18) en au moins une zone de ponction de la gaine (28) ; et
- dans les parties d'extrémité (26), la face interne de la bague est en contact de liaison mécanique avec la surface de la gaine (20), avec interposition du matériau isolant de la gaine entre le conducteur (18) et la bague (22) dans des régions (30) situées de part et d'autre de ladite au moins une zone de ponction de la gaine,
- le conducteur (18) est un microcâble de coeur plein et qui est entouré par la gaine (20) en matériau polymère électriquement isolant ;
- la bague est disposée sur le microcâble dans une région essentiellement non dénudée de celui-ci mais comprenant ladite au moins une zone de ponction de la gaine, **caractérisée en ce que**, la sonde étant fabricable par mise en oeuvre du procédé selon la revendication 1
- dans la partie centrale (24), le matériau isolant repoussé est interposé entre la bague et le microcâble dans des zones (30) adjacentes à ladite au moins une zone de ponction de la gaine.

3. La sonde de la revendication 2, dans laquelle ladite au moins une zone de ponction de la gaine est une zone circonférentielle continue et située dans un plan orthogonal à l'axe de la gaine.

4. La sonde de la revendication 2, dans laquelle ladite au moins une zone de ponction de la gaine est une zone circonférentielle discontinue et située dans un plan orthogonal à l'axe de la gaine, avec une pluralité de zones (44) de ponction de la gaine ménageant entre elles des zones (46) de continuité de l'isolant reliant les deux parties de gaine situées de part et d'autre de la bague.

5. La sonde de la revendication 2, dans laquelle la bague comprend sur sa face interne au moins un relief apte à définir une zone de ponction de la gaine correspondante.

6. La sonde de la revendication 5, dans laquelle ledit relief est un relief comprenant un profil longitudinal homologue de celui d'un mors de sertissage, sans surépaisseur de la bague à l'endroit de ce relief.

7. La sonde de la revendication 5, dans laquelle ledit relief comprend une surépaisseur (40, 42, 48) formée sur la face interne ou externe de la bague.

8. La sonde de la revendication 5, dans laquelle, en direction longitudinale, ledit relief s'arrête avant l'extrémité de la bague.

9. La sonde de la revendication 5, dans laquelle la bague porte sur sa face interne une pluralité de cannelures (34, 36) comprenant des parties rectilignes, des parties hélicoïdales (34) et/ou des parties longitudinales (36).

10. La sonde de la revendication 2, dans laquelle la bague porte sur sa face externe des nervures longitudinales de renfort (38).

11. La sonde de la revendication 2, dans laquelle l'épaisseur de paroi de la bague est comprise entre 20 et 100 µm.

12. La sonde de la revendication 2, dans laquelle la longueur de la bague est comprise entre 0,5 et 1,5 mm.

13. La sonde de la revendication 2, dans laquelle le matériau de la bague est un alliage de platine iridié ou de palladium, et le matériau de surface du microcâble est un alliage de type MP35N ou MP35NLT.

14. La sonde de la revendication 2, dans laquelle l'épaisseur de paroi de la gaine est comprise entre 5 et 50 µm.

15. La sonde de la revendication 2, dans laquelle le matériau polymère électriquement isolant de la gaine est choisi dans le groupe suivant : polyuréthanes (PU), polyesters (PET), polyamides (PA), polycarbonates (PC), polyimides, polymères fluorés, polyéther-éther-cétone (PEEK), poly-p-xylylène (parylène), polyméthacrylate de méthyle (PMM), PTFE (polytétrafluoroéthylène), FEP (propylène perfluoré), PFA (résine de copolymère perfluoroalkoxy), THV (tétrafluoroéthylène, hexafluoropropylène, fluorure de vinylidène), PVDF (polyfluorure de vinylidène), EFEP (éthylène propylène éthylène fluoré) et ETFE (éthylène tétrafluoroéthylène).

## Patentansprüche

1. Verfahren zur Herstellung einer Detektions-/Stimulationssonde, die dazu bestimmt ist, in ein venöses, arterielles oder lymphatisches Netz implantiert zu werden, wobei dieses Verfahren die folgenden Schritte aufweist:
- Bereitstellung eines Leiters (18) mit einem Mantel (20) aus elektrisch isolierendem Polymermaterial, welcher den Leiter auf seinem Umfang und auf seiner Länge bedeckt, wobei der Außendurchmesser des Ganzen höchstens gleich 2 French (0,66 mm) ist;
- Bereitstellung mindestens einer Detektions-/Stimulationselektrode, die von einer Metallhülse (22) gebildet wird, wobei die Hülse zwei Endteile (26) aufweist, die sich in Längsrichtung beiderseits eines mittleren Teils (24) erstrecken; und
- Herstellung einer Kontaktstelle des Leiters mit der Hülse, wobei
- der Schritt der Herstellung einer Kontaktstelle des Leiters mit der Hülse ein Crimpen der Hülse (22) auf den Leiter (18) aufweist, wobei dieser Leiter ein Mikrokabel mit massivem Kern ist, der von dem Mantel aus elektrisch isolierendem Polymermaterial umgeben ist,
- das Crimpen in einem im Wesentlichen nicht abisolierten Bereich des Mikrokabels durchgeführt wird, und
- das Crimpen in dem mittleren Teil (24) der Hülse durchgeführt wird,
wobei das Verfahren **dadurch gekennzeichnet ist, dass** das Crimpen wie folgt durchgeführt wird:
(i) Festklemmen des Mantels zwischen der Hülse und dem Mikrokabel,
(ii) lokales Durchbohren der Dicke des Mantels mit einem Bereich der Innenseite der Hülse, derart, dass in wenigstens einem Punktionsbereich (28) des Mantels die Hülse mit dem Mikrokabel in einen mechanischen und elektrischen Verbindungskontakt gebracht wird; und
(iii) Zurückschieben des isolierenden Materials in Bereichen, die dem Punktionsbereich des Mantels benachbart sind,
wobei das zurückgeschobene isolierende Material des Mantels in Bereichen (30), die sich beiderseits des Punktionsbereiches des Mantels befinden, zwischen dem Mikrokabel und der Hülse angeordnet bleibt.

2. Detektions-/Stimulationssonde, die dazu bestimmt ist, in ein venöses, arterielles oder lymphatisches Netz implantiert zu werden, wobei diese Sonde aufweist:
- einen Leiter (18), der geeignet ist, mit einem Generator eines aktiven implantierbaren medizinischen Geräts verbunden zu werden;
- einen Mantel (20) aus elektrisch isolierendem Polymermaterial, welcher den Leiter auf seinem Umfang und auf seiner Länge bedeckt; und
- mindestens eine Detektions-/Stimulationselektrode, die von einer mit dem Leiter elektrisch verbundenen Metallhülse (22) gebildet wird und dazu bestimmt ist, mit der Wand eines Zielgefäßes des venösen, arteriellen oder lymphatischen Netzes in Kontakt zu kommen,
und wobei:
- der Außendurchmesser der Sonde höchstens gleich 2 French (0,66 mm) ist;
- die Hülse zwei Endteile (26) aufweist, die sich in Längsrichtung beiderseits eines mittleren Teils (24) erstrecken;
- in dem mittleren Teil (24) die Innenseite der Hülse sich in mindestens einem Punktionsbereich (28) des Mantels in einem mechanischen und elektrischen Verbindungskontakt mit der Oberfläche des Leiters (18) befindet; und
- in den Endteilen (26) die Innenseite der Hülse sich in einem mechanischen Verbindungskontakt mit der Oberfläche des Mantels (20) befindet, mit Anordnung des isolierenden Materials des Mantels zwischen dem Leiter (18) und der Hülse (22) in Bereichen (30), die sich beiderseits des mindestens einen Punktionsbereiches des Mantels befinden,
- der Leiter (18) ein Mikrokabel mit massivem Kern ist, das von dem Mantel (20) aus elektrisch isolierendem Polymermaterial umgeben ist;
- die Hülse auf dem Mikrokabel in einem im Wesentlichen nicht abisolierten Bereich desselben, welcher jedoch den mindestens einem Punktionsbereich des Mantels aufweist, angeordnet ist,
**dadurch gekennzeichnet, dass**, wenn die Sonde durch Durchführung des Verfahrens nach Anspruch 1 herstellbar ist,
- in dem mittleren Teil (24) das zurückgeschobene isolierende Material in Bereichen (30), die dem mindestens einem Punktionsbereich des Mantels benachbart sind, zwischen der Hülse und dem Mikrokabel angeordnet ist.

3. Sonde nach Anspruch 2, wobei der mindestens eine Punktionsbereich des Mantels ein zusammenhängender und in einer zur Achse des Mantels orthogonalen Ebene befindlicher Bereich ist.

4. Sonde nach Anspruch 2, wobei der mindestens eine Punktionsbereich des Mantels ein nicht zusammenhängender und in einer zur Achse des Mantels orthogonalen Ebene befindlicher Bereich ist, mit mehreren Punktionsbereichen (44) des Mantels, zwischen denen zusammenhängende Bereiche (46) des Isoliermaterials angeordnet sind, welche die zwei beiderseits der Hülse befindlichen Mantelteile verbinden.

5. Sonde nach Anspruch 2, wobei die Hülse auf ihrer Innenseite mindestens eine Erhebung aufweist, die geeignet ist, einen Punktionsbereich des entsprechenden Mantels zu definieren.

6. Sonde nach Anspruch 5, wobei die Erhebung eine Erhebung ist, welche ein Längsprofil aufweist, das demjenigen eines Crimp-Stempels entspricht, ohne Überdicke der Hülse am Ort dieser Erhebung.

7. Sonde nach Anspruch 5, wobei die Erhebung eine Überdicke (40, 42, 48) aufweist, die auf der Innen- oder Außenseite der Hülse ausgebildet ist.

8. Sonde nach Anspruch 5, wobei in Längsrichtung die Erhebung vor dem Ende der Hülse endet.

9. Sonde nach Anspruch 5, wobei die Hülse auf ihrer Innenseite mehrere Rillen (34, 36) trägt, die geradlinige Teile, spiralförmige Teile (34) und/oder längs verlaufende Teile (36) aufweisen.

10. Sonde nach Anspruch 2, wobei die Hülse auf ihrer Außenseite längs verlaufende Verstärkungsrippen (38) trägt.

11. Sonde nach Anspruch 2, wobei die Wanddicke der Hülse zwischen 20 und 100 µm liegt.

12. Sonde nach Anspruch 2, wobei die Länge der Hülse zwischen 0,5 und 1,5 mm liegt.

13. Sonde nach Anspruch 2, wobei das Material der Hülse eine Platin-Iridium- oder Palladium-Legierung ist und das Oberflächenmaterial des Mikrokabels eine Legierung vom Typ MP35N oder MP35NLT ist.

14. Sonde nach Anspruch 2, wobei die Wanddicke des Mantels zwischen 5 und 50 µm liegt.

15. Sonde nach Anspruch 2, wobei das elektrisch isolierende Polymermaterial des Mantels aus der folgenden Gruppe ausgewählt ist: Polyurethane (PU), Polyester (PES), Polyamide (PA), Polycarbonate (PC), Polyimide, fluorierte Polymere, Polyetheretherketon (PEEK), Poly-p-xylylen (Parylene), Polymethylmethacrylat (PMMA), PTFE (Polytetrafluorethylen), FEP (Perfluorpropylen), PFA (Perfluoralkoxy-Copolymer)-Harz, THV (Tetrafluorethylen, Hexafluorpropylen, Vinylidenfluorid), PVDF (Vinylidenpolyfluorid), EFEP (fluoriertes Ethylen-Propylen-Ethylen) und ETFE (Ethylen-Tetrafluorethylen).

## Claims

1. A method for manufacturing a detection/stimulation lead intended to be implanted in a venous, arterial or lymphatic system,
the method comprising the following steps:
- obtaining a conductor (18) with a sheath (20) made of an electrically insulating polymer material covering the conductor over its periphery and over its length, the outer diameter of the whole being at most equal to 2 French (0.66 mm) ;
- obtaining at least one detection/stimulation electrode formed by a metal ring (22), the ring comprising two end parts (26) extending longitudinally on either side of a central part (24); and
- making a contact between the conductor and the ring, wherein
- the step of making a contact between the conductor and the ring comprises a crimping of the ring (22) to the conductor (18), this conductor being a solid core micro-cable that is surrounded by said sheath of electrically insulating polymer material,
- the crimping is performed in a substantially non-naked region of the micro-cable, and
- the crimping is operated in the central part (24) of the ring,
the method being **characterized in that** the crimping is operated so as to:
i) squeeze the sheath between the ring and the micro-cable,
ii) locally pierce the thickness of the sheath with an area of the inner face of the ring, so as to place the ring into mechanical and electrical connection contact with the micro-cable in at least one puncture area (28) of the sheath, and
iii) chase the insulating material in areas adjacent to said puncture area of the sheath,
wherein the chased insulating material of the sheath remains interposed between the micro-cable and the ring in regions (30) located on either side of said puncture area of the sheath.

2. A detection/stimulation lead intended to be implanted in a venous, arterial or lymphatic system, the lead comprising:
- a conductor (18) adapted to be connected to a generator of an active implantable medical device;
- a sheath (20) made of an electrically insulating polymer material, covering the conductor over its periphery and over its length; and
- at least one detection/stimulation electrode formed by a metal ring (22) electrically connected to the conductor, intended to come into contact with the wall of a target-vessel of said venous, arterial or lymphatic system,
and in which:
- the outer diameter of the lead is at most equal to 2 French (0.66 mm);
- the ring comprises two end parts (26) extending longitudinally on either side of a central part (24);
- in the central part (24), the inner face of the ring is in mechanical and electrical connection contact with the surface of the conductor (18) in at least one puncture area of the sheath (28); and
- in the end parts (26), the inner face of the ring is in mechanical connection contact with the surface of the sheath (20), with interposition of the insulating material of the sheath between the conductor (18) and the ring (22) in regions (30) located on either side of said at least one puncture area of the sheath;
- the conductor (18) is a solid core micro-cable that is surrounded by the sheath (20) of electrically insulating polymer material;
- the ring is placed on the micro-cable in a region of the latter that is essentially non-naked but that comprises said at least one puncture area of the sheath,
**characterized in that**, the lead being able to be manufactured by implementation of the method according to claim 1,
- in the central part (24), the chased insulating material is interposed between the ring and the micro-cable in areas (30) adjacent to said at least one puncture area of the sheath.

3. The lead of claim 2, wherein said at least one puncture area of the sheath is a continuous circumferential area that is located in a plane orthogonal to the axis of the sheath.

4. The lead of claim 2, wherein said at least one puncture area of the sheath is a discontinuous circumferential area that is located in a plane orthogonal to the axis of the sheath, with a plurality of puncture areas (44) of the sheath forming between them areas (46) of continuity of the insulator connecting the two sheath parts located on either side of the ring.

5. The lead of claim 2, wherein said the ring comprises on its inner face at least one relief adapted to define a corresponding puncture area of the sheath.

6. The lead of claim 5, wherein said relief is a relief comprising a longitudinal profile homologous to that of a crimping jaw, without extra thickness of the ring at the place of this relief.

7. The lead of claim 5, wherein said relief comprises an extra thickness (40, 42, 48) formed on the inner or outer face of the ring.

8. The lead of claim 5, wherein, in the longitudinal direction, said relief ends before the end of the ring.

9. The lead of claim 5, wherein the ring carries on its inner face a plurality of grooves (34, 36) comprising rectilinear parts, helical parts (34) and/or longitudinal parts (36).

10. The lead of claim 2, wherein the ring carries on its outer face longitudinal reinforcing ribs (38).

11. The lead of claim 2, wherein the wall thickness of the ring is comprised between 20 and 100 µm.

12. The lead of claim 2, wherein the length of the ring is comprised between 0.5 and 1.5 mm.

13. The lead of claim 2, wherein the material of the ring is a platinum iridium or palladium alloy, and the surface material of the micro-cable is an alloy of the MP35N or MP35NLT type.

14. The lead of claim 2, wherein the wall thickness of the sheath is comprised between 5 and 50 µm.

15. The lead of claim 2, wherein the electrically insulating polymer material of the sheath is chosen in the following group: polyurethanes (PU), polyesters (PET), polyamides (PA), polycarbonates (PC), polyimides, fluorinated polymers, polyether-ether-ketone (PEEK), poly-p-xylylene (parylene), poly(methyl methacrylate) (PMM), PTFE (polytetrafluroethylene), FEP (perfluorinated propylene), PFA (perfluoroalkoxy copolymer resin), THV (tetrafluoroethylene, hexafluoropropylene, vinylidene fluoride), PVDF (poly(vinylidene fluoride)), EFEP (fluorinated ethylene-propylene-ethylene) and ETFE (ethylene-tetrafluorethylene).
